# EUROPEAN PATENT SPECIFICATION

(11) **EP 1 829 536 B1**
(45) Date of publication and mention of the grant of the patent: **30.03.2011**
(21) Application number: 05809752.8
(22) Date of filing: 25.11.2005
(51) Int. Cl.: A61K 31/122, A61P 3/10, A61P 13/12, A61P 19/06

(54) **AMELIORANT FOR RENAL INSUFFICIENCY**
MITTEL ZUR LINDERUNG VON NIERENINSUFFIZIENZ
AGENT SOULAGEANT L'INSUFFISANCE RENALE

(30) Priority: 25.11.2004 JP 2004341019
(43) Date of publication of application: 05.09.2007
(73) Proprietor: Meiji Dairies Corporation, Koto-ku, Tokyo 136-8908 (JP)
(72) Inventor: SATO, Keisuke c/o Tottori University Faculty of Medicine, Tottori (JP); SAITO, Motoaki c/o Tottori University Faculty of Medicine, Tottori (JP); LUU, Bang c/o CNRS, Universite Louis Pasteur, 67084 Strasbourg (FR); YAMADA, Masashi c/o Meiji Dairies Corporation, Tokyo (JP); SUZUKI, Hiroto c/o Meiji Dairies Corporation, Tokyo (JP)
(74) Representative: HOFFMANN EITLE
(86) International application number: PCT/JP2005/021705
(87) International publication number: WO 2006/057346

(56) References cited:
- EP-A- 0 031 727
- WO-A-02/29014
- WO-A-02/066023
- WO-A-02/066024
- JP-A- 2000 083 623
- JP-A- 2002 241 270
- JP-A- 2002 517 738
- JP-A- 2004 000 115
- Nanzando, Igaku Daijiten Gokoban, 18th edition, 16 Januari, 1988, page 1488, "Tonyobyosei Jinsho" XP002997526

## Description

### Technical Field

The present invention relates to a non peptide low molecular weight compound which improves renal dysfunction caused by diabetes,

### Background Art

Renal dysfunction is caused by acute nephritis or chronic nephritis, diabetes, gout, side effect of medicaments, or the like and means a condition that filtering function of the kidney is lowered and internal circulation is disordered. However, there is no medicament for treating the renal dysfunction and thus it has been coped with dietary restriction and improvement of lifestyle. In the case of patients with advanced pathology, dialysis or renal transplantation is carried out. Renal dysfunction is a serious disease which remarkable deterioration quality of life (QOL) of patients.

The number of patients who are treated with dialysis in Japan increases year after year, which is, for example, 47,978 patients at the end of 1982, 123,925 patients at the end of 1992, and 229,538 patients at the end of 2002 (http://www.maeda-hospital.org/30thNovember2003.htm). The number of renal transplantation is 10,171 persons in USA, 1,749 persons in France, 1,768 persons in England, and 524 persons in Japan and the renal transplantation has been carried out in other Asian countries. In particular, very large number of cases of renal transplantation is carried out in China and India, amounting to 1,900 persons and 2,200 persons, respectively (http;//www.medi-net.or jp/tcnet/tc_1/1_3.html).

The renal dysfunction sometimes develops from diabetes, hypertension, or systemic lupus erythematosus as a causal disease. Particularly, diabetic renal disease is rapidly increasing in recent years according to increase of diabetic patients (*"kanja" Shiten kara* no *Approach* 2004, published by Testa Marketing).

WO 02/066023 A1 describes the use of compounds of the following formula (I), wherein R¹, R² and R³ each independently represents a hydrogen atom or a methyl group .and X represents a linear or branched C₁₀₋₂₈ alkylene or alkenylene group for diabetes complications, such as diabetic neuropathy.

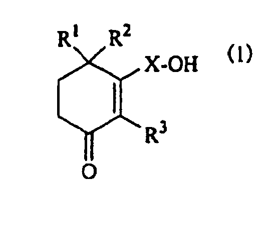

EP 0 031 727 A2 discloses the use of compounds of the formula: 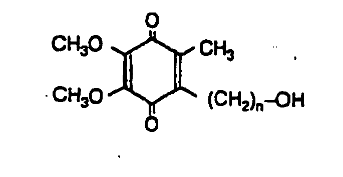 wherein n is an integer of from 4 to 22 for treating an ischemic disease such as cerebral apoplexy, cardiac insufficiency, or renal insufficiency due to hypertensive vascular lesions.

### Disclosure of the Invention

### Problems to be Solved by the Invention

An object of the present invention is to provide a low molecular weight compound which improves renal dysfunction caused by diabetes.

### Means for Solving the Problems

It has already been reported that a cyclohexenone long-chain alcohol has a neurotrophic activity which promotes survival of neurons and development of neurites (Gonzales de Aguilar et al., Brain Res (2001) 920, 65-73).

Furthermore, it has been demonstrated that the compound is useful as an agent for treating diabetic complications (JP-A-2002-241270) and as an agent for treating dysuria (JP-A-2002-241271).

Now, as a result of the extensive studies for solving the above problems, the inventors of the present invention have found that a cyclohexenone long-chain alcohol represented by the following formula (1) improves renal dysfunction and thus have accomplished the present invention.

Namely the invention provides an agent for improving renal dysfunction caused by diabetes comprising as an active ingredient a compound represented by the following formula (1): 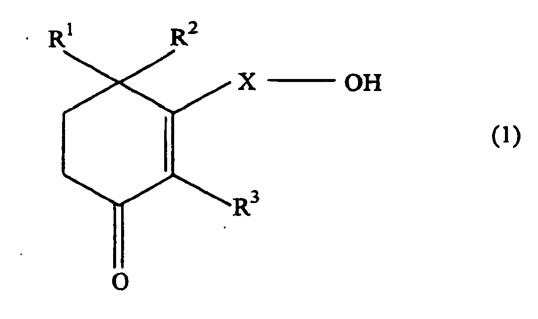 wherein each R¹, R², and R³ represents a hydrogen atom or a methyl group, and X represents a linear or branched alkylene or alkenylene group having 10 to 28 carbon atoms.

### Effect of the Invention

The compound represented by the formula (1) improves various parameters of renal function in model animals with renal dysfunction and is also useful as an agent for preventing and/or treating renal dysfunction. Thus, it can be expected that the compound remarkably improves the quality of life (QOL) of patients with renal dysfunction.

### Best Mode for Carrying Out the Invention

In the present invention, the renal dysfunction means a condition which is caused by diabetes, and exhibits symptoms of lowered filtering function of the kidney, disorder of the internal circulation, or the like. Specific examples thereof include glomerulonephritis (acute glomerulonephritis, rapidly progressive glomerulonephritis, chronic glomerulonephritis, membranoproliferative glomerulonephritis), chronic nephritis, secondary nephritis, nephrotic syndrome, uremic toxin, renal failure (acute renal failure, chronic renal failure), diabetic renal disease, hypertensive renal damage (nephrosclerosis), pyelonephritis, gouty kidney, interstitial nephritis, nephrosclerosis, multiple cystic kidney, renal lithiasis (urinary lithiasis, renal lithiasis), renal tumor (renal cell carcinoma, renal pelvic carcinoma), and the like.

In the above formula (1), X represents a linear or branched alkylene or alkenylene group having 10 to 28 carbon atoms, preferably 10 to 18 carbon atoms. The side chain of the branched alkylene or alkenylene group includes an alkyl group having 1 to 10 carbon atoms. Examples of the side-chain alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, an isohexyl group, a heptyl group, an octyl group, a nonyl group, a decyl group, and the like. Among these, a methyl group is particularly preferable. Moreover, the substitution of the side chain to a linear alkylene group or a linear alkenylene group (which means an alkene structure having at least one carbon-carbon double bond) is preferably carried out at the 3- and/or 7-position. Among these Xs, a linear alkylene group having 10 to 28 carbon atoms is more preferable, and a linear alkylene group having 10 to 18 carbon atoms is particularly preferable. Furthermore, R¹, R², and R³ each represents a hydrogen atom or a methyl group. A case where at least one of them represents a methyl group is more preferable and a case where R¹, R², and R³ each represents a methyl group is particularly preferable.

The compounds represented by the formula (1) may be in a form of a pharmaceutically acceptable salt thereof, or a solvate or hydrate thereof. There may be various isomers of the compound represented by the formula (1) and these isomers are also included in the present invention.

The compound represented by the formula (1) can be prepared by known methods. For example, it can be produced in accordance with the production process described in JP-A-2000-297034.

The compound represented by the formula (1) can be administered to mammals such as human through either an oral route or a parenteral route such as intramuscular, subcutaneous or intravenous injection, or a suppository. Moreover, it is also possible to administer the agent of the present invention for improving renal dysfunction in combination with agents for treating diseases such as diabetes and gout. Furthermore, the agent of the present invention for improving renal dysfunction can be also used as an agent for preventing renal dysfunction for patients of diabetes, gout, and the like.

When oral preparations are prepared, the compound is formulated into tablets, coated tablets, granules, capsules, solutions, syrups, elixirs, oily or aqueous suspensions in a usual manner after the addition of an excipient and if necessary, a binder, a disintegrator, a lubricant, a colorant and/or a corrigent. Examples of the excipient include lactose, corn starch, saccharose, glucose, sorbit, crystalline cellulose, and the like. Examples of the binder include polyvinyl alcohol, polyvinyl ether, ethyl cellulose, methyl cellulose, gum arabic, tragacanth, gelatin, shellac, hydroxypropyl cellulose, hydroxypropyl starch, polyvinyl pyrrolidone, and the like.

Examples of the disintegrator include starch, agar, gelatin powder, crystalline cellulose, calcium carbonate, sodium hydrogen carbonate, calcium citrate, dextran, pectin, and the like. Examples of the lubricant include magnesium stearate, talc, polyethylene glycol, silica, hardened vegetable oils, and the like. As the colorant, those permitted as additives for pharmaceuticals can be used. As the corrigent, cocoa powder, menthol, aromatic acid, peppermint oil, camphol, cinnamon powder, and the like can be used. The tablets and granules may be coated with sugar, gelatin, or other coatings as needed.

When injections are prepared as examples of parenteral administration, a pH regulator, buffer, stabilizer and/or preservative are added if necessary, followed by formulation into subcutaneous, intramuscular, or intravenous injection in a usual manner. The injection may be formulated into a preparation to be reconstituted immediately before use by filling the solution in a container and lyophilizing it into a solid preparation. Moreover, a single dose may be filled in a container or multiple doses may be filled in one container.

In the case of human, the dose of the compound of the invention as a medicament is usually in the range of 0.01 to 1000 mg/day, preferably 0.1 to 500 mg/day per adult. The daily dose is administered once a day or in 2 to 4 portions a day.

### Examples

Although the following will explain the present invention with reference to Examples, the present invention is not limited to these Examples.

### Example 1

To 8 weeks old male SD rats, 50 mg/kg of STZ (streptozotocin, manufactured by Wako Pure Chemical Industries, Ltd.) was intraperitoneally administered, thereby inducing diabetes. The diabetes model rats were divided into four groups: as therapeutic groups, 2,4,4-trimethyl-3(15-hydroxypentadecyl)-2-cyclohexen-1-one (Compound 1: 2, 8 mg/kg) synthesized by a method described in JP-A-2000-297034 was intraperitoneally administered once a day continuously for 4 or 8 weeks; and as controls, a non-therapeutic group with diabetes alone (Diabetic Control) and a control group without treatment were prepared. After 4 or 8 weeks, serum and both kidneys were removed from the rats. The right kidney was stored in a frozen state and the left kidney was subjected to formalin fixation.

Increase of kidney weight, elevation of serum creatinine level, and increase of MDA in kidney may be observed by renal dysfunction caused by diabetes. First, with regard to the kidney weight, in the rats to which the compound was administered, the increase of kidney weight was suppressed on the fourth week after STZ administration and, in the case of 8 mg/kg administration, it was markedly suppressed on the eighth week (Table 1).

With regard to serum creatinine level, in the group of 8 mg/kg administration, the elevation was markedly suppressed on the fourth week and on the eighth week after STZ administration (Table 2). In this connection, even in the group of 4 mg/kg administration, a tendency of the suppression was observed on the eighth week. With regard to serum urea nitrogen, the elevation was markedly suppressed on the fourth week after STZ administration in the group of 8 mg/kg administration (Table 3). With regard to MDA in kidney, the increase was markedly suppressed both in the group of 8 mg/kg administration and in the group of 2 mg/kg administration (Table 4).

Based on the above results, the compound is promising as an agent for improving renal dysfunction.

**Table 1**

| Weight of kidney g | | | |
|---|---|---|---|
| Four weeks after STZ administration, Eight weeks after STZ administration | | | |
| | | 4 weeks after STZ administration | 8 weeks after STZ administration |
| Control | | 1.21±0.02 | 1.30±0.03 |
| Diabetic Control | | 1.46±0.06* | 1.51±0.05* |
| Compound 1 | 2 mg/kg | 1.33±0.05* | 1.59±0.04* |
| Compound 1 | 8 mg/kg | 1.27±0.07 | 1.33±0.05** |

| | | | |
|---|---|---|---|
| *: significantly different from Control (5% or less compared with Control group, p<0.05) **: significantly different from Diabetic Control (5% or less compared with Diabetes group, p<0.05) | | | |

**Table 2**

| Serum creatinine mg/dL | | | |
|---|---|---|---|
| Four weeks after STZ administration, Eight weeks after STZ administration | | | |
| | | 4 weeks after STZ administration | 8 weeks after STZ administration |
| Control | | 0.865±0.088 | 1.335±0.058 |
| Diabetic Control | | 1.916±0.164* | 2.959±0.381* |
| Compound 1 | 2 mg/kg | 2.354±0.254* | 2.504±0.253* |
| Compound 1 | 8 mg/kg | 1.004±0.118** | 1.836±0.228** |

| | | | |
|---|---|---|---|
| *: significantly different from Control (5% or less compared with Control group, p<0.05) | | | |

**Table 3**

| Serum urea nitrogen mg/dL | | | |
|---|---|---|---|
| Four weeks after STZ administration, Eight weeks after STZ administration | | | |
| | | 4 weeks after STZ administration | 8 weeks after STZ administration |
| Control | | 28.31±1.95 | 33.40±1.34 |
| Diabetic Control | | 60.64±5.01* | 55.03±4.23* |
| Compound 1 | 2 mg/kg | 51.00±3.78* | 60.78±5.34* |
| Compound 1 | 8 mg/kg | 41.76±3.60** | 53.69±2.97** |

| | | | |
|---|---|---|---|
| *: significantly different from Control (5% or less compared with Control group, p<0.05) **: significantly different from Diabetic Control (5% or less compared with Diabetes group, p<0.05) | | | |

**Table 4**

| MDA in kidney mM/g tissue | | |
|---|---|---|
| Eight weeks after STZ administration | | |
| | | 8 weeks after STZ administration |
| Control | | 35.5±1.0 |
| Diabetic Control | | 63.4±1.3* |
| Compound 1 | 2 mg/kg | 56.1±3.2** |
| Compound 1 | 8 mg/kg | 55.7±2.0** |

| | | |
|---|---|---|
| *: significantly different from Control (5% or less compared with Control group, p<0.05) **: significantly different from Diabetic Control (5% or less compared with Diabetes group, p<0.05) | | |

While the present invention has been described in detail with reference to specific embodiments thereof, it will be apparent to one skilled in the art that various changes and modifications can be made therein without departing from the spirit and scope thereof.

The present application is based on Japanese Patent Application No. 2004-341019 filed on November 25, 2004, and the contents are incorporated herein by reference.

### Industrial Applicability

The compound represented by the formula (1) is useful as an excellent agent for improving renal dysfunction.

## Claims

1. An agent for use in a method for improving renal dysfunction caused by diabetes comprising as an active ingredient a compound represented by the following formula (1): 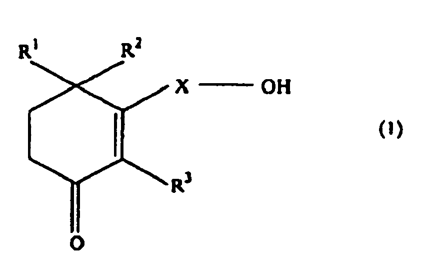 wherein each R¹, R², and R³ represents a hydrogen atom or a methyl group and X represents a linear or branched alkylene or alkenylene group having 10 to 28 carbon atoms.

2. The agent for use in a method for improving renal dysfunction according to claim 1, wherein X is a linear alkylene group having 10 to 28 carbon atoms.

3. The agent for use in a method for improving renal dysfunction according to claim 2, wherein X is a linear alkylene group having 10 to 18 carbon atoms.

4. The agent for use in a method for improving renal dysfunction according to any one of claims 1 to 3, wherein at least one of R¹, R², and R³ is a methyl group.

5. The agent for use in a method for improving renal dysfunction according to claim 1, wherein each R¹, R², R³ represents a methyl group.

6. Use of a compound represented by the following formula (1): 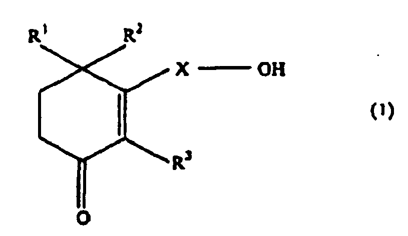 wherein each R¹, R², and R³ represents a hydrogen atom or a methyl group and X represents a linear or branched alkylene or alkenylene group having 10 to 28 carbon atoms, for the manufacture of an agent for improving renal dysfunction caused by diabetes.

## Patentansprüche

1. Mittel zur Verwendung in einem Verfahren zur Verbesserung der Nierendysfunktion, verursacht durch Diabetes, umfassend als aktiven Bestandteil eine Verbindung, dargestellt durch die folgende Formel (1): 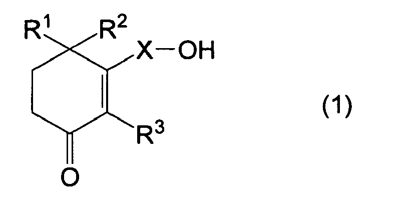 worin R¹, R² und R³ jeweils ein Wasserstoffatom oder eine Methylgruppe sind und X eine lineare oder verzweigte Alkylen- oder Alkenylengruppe mit 10 bis 28 Kohlenstoffatomen ist.

2. Mittel zur Verwendung in einem Verfahren zur Verbesserung der Nierendysfunktion gemäß Anspruch 1, worin X eine lineare Alkylengruppe mit 10 bis 28 Kohlenstoffatomen ist.

3. Mittel zur Verwendung in einem Verfahren zur Verbesserung der Nierendysfunktion gemäß Anspruch 2, worin X eine lineare Alkylengruppe mit 10 bis 18 Kohlenstoffatomen ist.

4. Mittel zur Verwendung in einem Verfahren zur Verbesserung der Nierendysfunktion nach einem der Ansprüche 1 bis 3, worin zumindest eines von R¹, R² und R³ eine Methylgruppe ist.

5. Mittel zur Verwendung in einem Verfahren zur Verbesserung der Nierendysfunktion nach Anspruch 1, worin R¹, R², R³ jeweils eine Methylgruppe sind.

6. Verwendung einer Verbindung, dargestellt durch die folgende Formel (1): 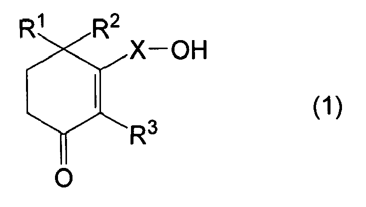 worin R¹, R² und R³ jeweils ein Wasserstoffatom oder eine Methylgruppe sind und X eine lineare oder verzweigte Alkylen- oder Alkenylengruppe mit 10 bis 28 Kohlenstoffatomen ist, zur Herstellung eines Mittels zur Verbesserung der Nierendysfunktion, verursacht durch Diabetes.

## Revendications

1. Agent pour utilisation dans un procédé destiné à améliorer le dysfonctionnement rénal provoqué par le diabète, comprenant comme principe actif un composé représenté par la formule (1) suivante : 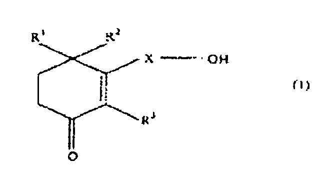 où chaque R¹, R² et R³ représentent un atome d'hydrogène ou un groupe méthyle et X représente un groupe alkylène ou alcénylène linéaire ou ramifié ayant de 10 à 28 atomes de carbone.

2. Agent pour utilisation dans un procédé destiné à améliorer le dysfonctionnement rénal selon la revendication 1, où X est un groupe alkylène linéaire ayant de 10 à 28 atomes de carbone.

3. Agent pour utilisation dans un procédé destiné à améliorer le dysfonctionnement rénal selon la revendication 2, où X est un groupe alkylène linéaire ayant de 10 à 18 atomes de carbone.

4. Agent pour utilisation dans un procédé destiné à améliorer le dysfonctionnement rénal selon l'une quelconque des revendications 1 à 3, où au moins un de R¹, R² et R³ est un groupe méthyle.

5. Agent pour utilisation dans un procédé destiné à améliorer le dysfonctionnement rénal selon la revendication 1, où chaque R¹, R² et R³ représentent un groupe méthyle.

6. Utilisation d'un composé représenté par la formule (1) suivante : 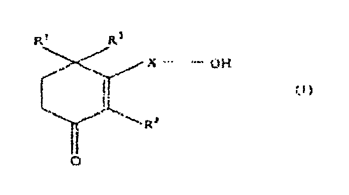 où chaque R¹, R² et R³ représentent un atome d'hydrogène ou un groupe méthyle et X représente un groupe alkylène ou alcénylène linéaire ou ramifié ayant de 10 à 28 atomes de carbone, pour la préparation d'un agent destiné à améliorer le dysfonctionnement rénal provoqué par le diabète.
